# EUROPEAN PATENT SPECIFICATION

(11) **EP 3 411 384 B1**
(45) Date of publication and mention of the grant of the patent: **22.04.2020**
(21) Application number: 17710391.8
(22) Date of filing: 31.01.2017
(51) Int. Cl.: C07K 1/34, C07K 14/52, C07K 14/54, C07K 1/36, C07K 14/475, A61K 35/20

(54) **EXTRACTION PROCESS FROM COLOSTRUM**
EXTRAKTIONSVERFAHREN AUS KOLOSTRUM
PROCÉDÉ D'EXTRACTION DU COLOSTRUM

(30) Priority: 04.02.2016 IT UB20160137
(43) Date of publication of application: 12.12.2018
(73) Proprietor: INNOMED S.A., 2350 Luxembourg (LU)
(72) Inventor: BRUNDO, Maria Violetta, 95021 Aci Castello (CT) (IT); SCIACCA, Salvatore, 95123 Catania (CT) (IT)
(74) Representative: Pistolesi, Roberto
(86) International application number: PCT/IB2017/050513
(87) International publication number: WO 2017/134559

(56) References cited:
- EP-A1- 0 410 272
- EP-A1- 2 762 490
- EP-A2- 1 055 372
- WO-A1-2013/098333
- P. SACERDOTE ET AL: "Biological components in a standardized derivative of bovine colostrum", JOURNAL OF DAIRY SCIENCE., vol. 96, no. 3, 1 March 2013 (2013-03-01), pages 1745-1754, XP055304556, US ISSN: 0022-0302, DOI: 10.3168/jds.2012-5928
- POULIOT Y ET AL: "Milk growth factors as health products: Some technological aspects", INTERNATIONAL DAIRY JOURNAL, ELSEVIER APPLIED SCIENCE, BARKING, GB, vol. 16, no. 11, 1 November 2006 (2006-11-01), pages 1415-1420, XP024963276, ISSN: 0958-6946, DOI: 10.1016/J.IDAIRYJ.2006.06.006 [retrieved on 2006-11-01]

## Description

The present invention relates to a method for obtaining a mixture of biological factors isolated from colostrum, characterized by one or more step of filtration and concentration that allow the separation of large proteins and pathogenic microorganisms from the permeate, and to maintain the biological factors of interest in the retentate.

The present invention further comprises a method for obtaining a mixture of biological factors comprising a reduced fraction of immunoglobulins or totally free of immunoglobulins, characterized by further steps of affinity chromatography and immunoaffinity.

### STATE OF THE ART

Stem cells are unspecialized primitive cells, with the ability to transform into different types of body cells through a process called cellular differentiation.

Stem cells are taken from different biological sources, such as umbilical cord, amniotic sac, blood, bone marrow, placenta, and adipose tissues, and they have been the subject of numerous studies since the early 60s for the treatment of various diseases, such as neurological disorders, ulcerative colitis, diabetes mellitus, diabetic foot, bone fractures, myocardial infarction, alopecia, tissue repair, etc.

Science has so far considered stem cells as totipotent entities capable of repairing any type of tissue injury, through differentiation. At the moment such developments are substantially far away from clinical practice due to ethical, religious, economic and, above all, technical issues. Moreover, it is not yet clear whether the reparative activity of stem cells is due to stem cells themselves, or to biological factors produced by the supernatant of such cells.

Already in 2006, it was speculated that stem cells did not transform into the injured tissue, but they would release biological factors responsible for injuries repair.

In the last decade, numerous publications (1-14) reported the presence in stem cells supernatant of biological substances or factors, such as growth factors and cytokines, capable of stimulating cellular repair.

In addition, several researchers obtained preliminary results, both *in vitro* and *in vivo,* on some pathologies using only the stem cell supernatant, free of the cellular component.

However, the use of such supernatant does not solve the ethical, economic and religious issues linked to this type of therapy, as the starting material to produce the supernatant is made, even so, from stem cells.

It should also be considered that, in clinical trials under way in recent years, the maximum therapeutic dose injected per patient is of 50 million stem cells, dose that produces approximately, depending on the type of culture, 55 ng of biological factors currently measurable. The reason for the poor clinical results obtained so far may, therefore, be due to the low concentration of biological factors secreted by such a small number of stem cells.

It is therefore apparent the need to have available growth factors suitable for use in the clinic, in an amount sufficient to carry out therapeutic activity with a certain effectiveness, and in the absence of regulatory, ethical, religious and economic type of issues.

It has been known for years that herbivore colostrum contains, to a greater extent than the omnivore one, several growth factors and cytokines, in addition, of course, to antibacterial aspecific factors, and immunoglobulins specific to some pathogens, that may be present depending on the species and breeds.

Moreover, various methods of extraction are known in the art to obtain some of these biological factors depending on the specific needs of different researchers, but no extraction method proposed up to now had the purpose of isolating all the biological factors known to be present in stem cell cultures, from an extract of colostrum, in a significantly higher concentration than that obtainable from the supernatant of stem cultures themselves; and in addition, depending on the therapeutic need, to keep or remove the immunoglobulin component.

These biological factors, common to the supernatant of stem cell cultures and colostrum of herbivorous mammals, are highly conserved in the phylogeny and, therefore, have a significant cross-reaction activity between humans and animals. Bovine colostrum results, thus, to be the richest source of biological factors, and therefore the most advantageous one.

Obviously, being the composition of the colostrum greatly different from the composition of the supernatant of human stem cells, the extraction method used must take into account the elimination of many factors, not only unnecessary but also potentially harmful to certain therapeutic uses, such as casein, lactose, furosine, fats in general, and obviously bacteria and viruses, while preserving the maximum possible concentration of various biological factors that mimic the activity of human stem cells.

The methods for extracting biological factors from colostrum known to date exhibit different types of issues, such as the presence of heat sterilization steps that lead to the loss of a large amount of biological factors in the final product, due thermal degradation, and the need of steps that provide for the elimination of casein, which can lead to allergic reactions. These steps require a coagulation/precipitation process followed by filtration at low pH values, which are the cause of the denaturation of many proteins, including several biological factors present in colostrum.

EP2762490 describes a method for obtaining immunoglobulins from colostrum.

Sacerdote et al., J Dairy Sci.), 96, 1745-1754 describe a method combining centrifugation and filtration for obtaining a standardized compositions of components from bovine colostrum. The patent application WO2011/064114 describes a process for obtaining a mixture of biological factors isolated from colostrum usable for nutraceutical formulations, based on an initial microfiltration step using a membrane with a molecular porosity comprised between 2 and 6 microns, a step for collecting the permeate containing the biological principles, an optional sterilizing filtration step of the permeate, and an optional step for removing the water contained in the permeate.

Several disadvantages were observed related to the use of the method described in said patent application.

First, the microfiltration step presents some elements of criticality, when applied to an heterogeneous product such as colostrum, because the efficiency of the process may be greatly reduced by the formation of lipid and casein colloids layers on the surface of the filter membrane, with consequent clogging of the membrane pores itself, and loss of biological factors that would thus be retained in the retentate

Moreover, this microfiltration step, following which the retentate is simply concentrated by tangential nanofiltration using a membrane having a molecular cut-off of 400 Da, leads to a poor reduction in lactose, as the molecular cut-off chosen is not suitable for the passage of the lactose in the permeate. In this case, thus, the finished product retains a certain content of lactose, with a clear high probability of formation of high concentrations of furosine, a potentially reactive intermediate present at the origin of colostrum, which is formed by means of a protein glycation process (reaction of Maillard) by the reaction of free reducing sugars, such as lactose, with proteins and amino acids subjected to heating. For this reason the upper limits for furosine in raw milk are set by law.

The method described in the prior art does not therefore include any steps that would prevent the formation of furosine or involving its elimination.

A similar process of extraction from colostrum is described in the patent applications WO2013/098 331 and WO2013/98333.

It is therefore evident the need to have available an industrially scalable method for extracting biological factors from bovine colostrum, which allows to obtain the biological factors suitable for use in the clinic, in an amount sufficient to carry out therapeutic activity with a certain effectiveness, and in the absence of regulatory, ethical, religious, and economic type of issues.

In particular, there is the evident need for the scientific and medical community to obtain a sterile mixture of biological factors derived from colostrum, that comprises cytokines, various growth factors, and in the presence or not of immunoglobulins, usable for alimentary purposes, therapeutic (topical and/or injection route) purposes, and in laboratory cultures, and that also has the advantages to be obtained by an industrially scalable process, characterized by an excellent reproducibility, cost-effective, and in line with the current regulatory requirements

### DEFINITIONS

Unless otherwise defined, all terms of the art, notations, and other scientific terms used herein are intended to have the meanings commonly understood by those skilled in the art to which this description belongs. In some cases, terms with meanings that are commonly understood are defined herein for clarity and/or ready reference; therefore, the inclusion of such definitions herein should not be interpreted as being representative of a substantial difference with respect to what is generally understood in the art.

According to the present invention, by "skimming" it is meant a separation operation of the lipid component from the colostrum by means of centrifugation at less than 10,000 rpm, and at a controlled temperature.

According to the present invention by "ultrafiltration" it is meant a filtration process operated using a semi-permeable anisotropic membrane characterized by a size of the pores in the order of magnitude of nanometers.

According to the present invention, by "diafiltration" it is meant a concentration process for the qualitative improvement of the ultrafiltrate, through the removal of water and low molecular weight components.

By "affinity chromatography" it is meant a chromatographic technique that is based on the interactions that are formed between a substance and the related ligand.

By "immunoaffinity chromatography" it is meant a chromatographic technique that is based on the selective binding between antigen and antibody.

By "tangential filtration" it is meant a filtration method where the flow of the fluid to be filtered flows tangentially to the surface of the filter in such a way that the filtrated sediment slides on the filtering surface, provided that it has a sufficiently low porosity so as not to effect the filtration among the spongy inner retaining mesh but on the contact face.

### DESCRIPTION OF THE INVENTION

An object of the present invention is to provide a method for extracting biological factors from colostrum, comprising the following steps:
a) skimming of colostrum, previously diluted with water or saline solution;
b) casein separation;
c) diafiltration using a polymeric membrane;
d) sterilizing filtration,
   wherein, in step a) the skimming is performed by centrifugation at 5,000-10,000 rpm, preferably at about 8,000 rpm, at a temperature comprised between 35 and 36°C under stirring. In this step of the process, the abatement of the lipid component of colostrum is realized by using the centrifugal force.

In this invention, casein separation step b) is performed using 0,1 µm ceramic filters, optionally preceded by 1.5 µm and 0.5 µm pre-filters. In this way it is possible to remove the casein without having to resort to pH changes that may inactivate the delicate colostrum components.

In a preferred aspect of the present invention, the permeate diafiltration step is performed using a polymeric membrane, preferably a 2,000-5,000 Da polymeric membrane, more preferably of about 3,000 Da.

In a further preferred aspect, the above polymeric membrane is a polysulfone membrane, or a polyethersulfone membrane.

In this case, the capacity of the ultrafiltration membrane with a molecular cut-off of 3,000 to allow the passage of the lactose in the permeate, and retain the protein component in the retentate, is exploited. Moreover, in this phase a concentration of the permeate, obtained by the previous tangential filtration step of casein removal, of about 10-20 times is obtained.

In a further aspect, the retentate obtained by the diafiltration process undergoes a sterilizing filtration step d) performed using a filter with a porosity of 0.22-0.45 µm, and subsequently with a filter with porosity of about 0.1 µm.

This filtration step allows to eliminate potential contaminants from the retentate obtained by the method of the present invention, and allows to obtain a mixture of active biological factors, and completely sterile from the microbiological point of view, safely usable for different human applications and uses.

In a preferred aspect of the present invention, the product obtained by the sterilizing filtration process is subjected to a lyophilization step.

Freeze-drying, which is performed in a controlled environment, generally at a maximum room temperature of 25-30°C, is a particularly useful and delicate process to remove water from a product, without risk of denaturing the components. Furthermore, freeze-drying is the most cost-effective solution that allows to obtain a lyophilized mixture of biological factors isolated from colostrum, which is easy to handle compared to a sterile solution, as it gives rise to a powder product, completely soluble, which can be stored at room temperature for a long time, and that can be easily shipped without special precautions, advantages which entail a considerable reduction in costs.

The method object of the present invention is, thus, a safe and effective method which allows eliminating the lipid component, casein and high molecular weight molecules from colostrum, while maintaining the biological factors present in colostrum unaltered and in a high concentration.

The product obtained by the extraction method object of the present invention, is characterized by a mixture of biological factors comprising also immunoglobulins specific to the species, which can be used by alimentary route or for therapeutic use (by topical administration).

In particular, the method described in the present invention offers the advantage of obtaining a mixture of factors isolated from bovine colostrum in a biologically active form, due to the fact that no treatments which may cause denaturation of the proteins and modify their tertiary structure, such as high temperatures or low pH values, are used.

This concept is of fundamental importance, because in order to perform their physiological functions in the body, these active factors cannot be in the denatured form, as the loss of the structure corresponds to the loss of effectiveness.

At the end of the extraction, the product is assessed for its qualitative and quantitative content of biological factors common to those present in the supernatant of stem cells, and for the biological and therapeutic activity of these factors both *in vitro* and *in vivo.*

A peculiar problem that was addressed is the use of preservatives, not so much to prevent any bacterial contaminations, which are nevertheless removed by the extraction steps, but to avoid the presence of endotoxins, toxic substances present in the outer membrane of Gram-negative bacteria which are impossible to remove from the finished product.

It is known in the art that, in pharmaceutical preparations, it is necessary to remove all traces of endotoxins that may involve the production process, because even small amounts may cause mild clinical effects of toxic type in patients treated with contaminated products.

One of the proposed solutions was to collect the colostrum into canisters containing some antibacterial products, such as phenoxyethanol and diazolidinyl urea, that prevent the proliferation of bacteria and, therefore, the development of endotoxins that are formed before the manufacturing process and are difficult to eliminate.

This solution is overcome by the present invention by carrying out the collection of colostrum in a controlled and protected environment, subjecting the collected material to rapid freezing by means of a blast chiller/freezer.

The main advantages of the present method for obtaining biological factors compared to the process described in the prior art (WO 2011/064114) are characterized by the fact that a higher concentration of biological factors in the finished product is obtained, with lower production costs

The method object of the present invention further comprises a step for the reduction of lactose, up to the maximum limit of 0.5% based on the dry substance, which allows to prevent furosine formation.

The extraction method of the present invention thus allows to obtain a remarkable decrease in the content of contaminants, such as casein, lactose and furosine, compared to the methods known in the art, with the advantage of minimizing the presence of lipid and carbohydrate substances not necessary for the described activity, and eliminating the formation during the process of molecules initially not contained in colostrum, avoiding in this way any allergenic effects.

A further advantage of the method of the present invention is related to the fact that the concentration of biological factors obtained following the above steps is higher than that present in the maximum therapeutic dose of stem cultures, usable only by injection.

The extract of biological factors obtained by the method of the present invention may therefore be used for alimentary use and for pharmaceutical use (topical route).

It is however known in the art that, for regulatory reasons, when biological factors are used by injection route, they cannot contain immunoglobulins. It is also known that, when the same biological factors are used for growing cell cultures *in vitro,* as a replacement for fetal calf serum (FCS), immunoglobulins must be present in very low concentration or, for some uses, they must be totally eliminated (for example for injection use).

Additional steps were then added to the method described above, that allow to obtain a product suitable for injection use, and therefore totally free of immunoglobulins, or to obtain a product usable as a growth factor for cell cultures, characterized in that immunoglobulins are reduced by 90%.

In both cases, the products obtained maintain a high biological factors activity increasing it by 60% because, at the same weight, the content of active factors is much higher.

A further object of the present invention thus comprises the method for colostrum purification described above, characterized by a further step of IgG depletion by affinity chromatography.

In a preferred aspect, in the method of the present invention affinity chromatography consisting of affinity columns selected from HiTrap™ (GE Healthcare Life Science) columns or HiScale™ (GE Healthcare Life Science) columns is used.

In a further aspect, the method described above is characterized by a further step of desalinization and concentration of the depleted sample.

In a further preferred aspect of the present invention, the above concentrated sample is subjected to a lyophilization process.

The above method therefore allows to remove IgG immunoglobulins from the mixture of biological factors described above by an amount higher than 90%, so as to obtain a mixture usable as a growth factor for cell cultures.

A further object of the present invention comprises a method for the total depletion of IgG, IgA e IgM immunoglobulins from the mixture of active factors obtained by the extraction process from colostrum described above, comprising the following steps:
a) affinity chromatography;
b) tangential flux filtration;
c) immunoaffinity chromatography.

According to the present invention, the affinity chromatography step a) allows a partial depletion of IgG and IgA.

According to the present invention, the filtration step b) allows a partial depletion of IgM and aggregates of immunological nature.

According to the present invention, the immunoaffinity step c) allows the complete elimination of IgG, IgA and IgM immunoglobulins.

The process according to the present invention thus allows to obtain a mixture of biological factors completely free of immunogenicity, usable for obtaining pharmaceutical formulations administrable by injection route.

In a preferred aspect of the present invention, the tangential flow filtration step makes use of membranes with a molecular cut-off of about 750 kDa/0.1 µm.

In a preferred aspect of the present invention, the IgG, IgA and IgM depletion method includes techniques based on affinity chromatography characterized by a stationary phase of protein nature with high selectivity towards immunoglobulins. The above stationary phases may be selected from Protein G, Protein A or Peptide M.

In a further preferred aspect of the present invention, said immunoaffinity chromatography step c) is characterized by the use of NHS-activated columns

According to a preferred aspect of the present invention, the above immunoaffinity chromatography method is followed by a step of desalinization and concentration of the depleted sample by diafiltration using membranes having a cut-off of 3000-5000 Da.

According to a further aspect of the present invention, the colostrum used is derived from humans or domestic animals, selected from the group consisting of bovine, ovine and equine, preferably bovine.

According to a further aspect of the present invention, the colostrum used is collected in the first hours after birth, preferably after six hours.

An object of the present invention is a combination of active factors obtained by the method of the present invention, comprising cytokines, growth factors, chemotactic factors, stem cell stimulating factors, complement proteins, antibacterial and/or antiviral factors, and immunoglobulins.

A further object of the present invention is a combination of active factors obtained by the method of the present invention including cytokines, growth factors, chemotactic factors, stem cell stimulating factors, complement proteins, and antibacterial and/or antiviral factors.

An object of the present invention is a combination of active factors obtained by the method of the present invention, comprising cytokines, growth factors, chemotactic factors, stem cell stimulating factors, complement proteins, antibacterial and/or antiviral factors, and immunoglobulins, characterized by having a lactose content lower than 1%.

In the combination obtained by the method of the present invention, the cytokines are selected from IL-1α, IL-1 β, IL-2, IL-4, IL-6, IL-8, IL-10, IL-12, IL-15,IL-17, IFN-γ, TNF-α, and IL-1Ra.

The growth factors are selected from TGF-β1, IGF-1, NGF, PDGF, EGF, BMP2, β-FGF, FGF-2, HGF, and VEGF.

The chemotactic factors are selected from eotaxin, MCP-1 (Monocyte Chemotactic Factor-1).

The stem cell stimulating factors are selected from G-CSF, GM-CSF, LIF (Leukemia Inhibitory Factor), SCF (Stem Cell Factor), and SDF-1 (Stromal Derived Factor-1). The complement proteins are selected from C3A and C4A.

The antibacterial and/or antiviral factors are selected from transferrin, lactoferrin, lysozyme, and lactoperoxidase.

The immunoglobulins are selected from IgG, IgA, and IgM.

### COLOSTRUM COLLECTION

Colostrum is collected from pluripara Holstain breed cows in the six hours after birth. The collection takes place in a protected environment, and in a nitrogen atmosphere, using gamma-sterilized containers immediately subjected to rapid freezing, in order to avoid the natural bacterial development due to high concentration of growth factors in the colostrum.

PROCESS STAGES (the processes of the invention are described in the claims. Any subject-matter falling outside the scope of the claims is provided for information purposes only).
- Skimming
- Casein separation using ceramic filters
- Concentration by diafiltration using a 3000 Da polymeric membrane.
- Filtration using a 0,45-0,22 µm cartridge filter and a 0,1 µm one (for injection use and cell cultures).
- Freezing at -20°C and/or lyophilization

### Skimming

At this stage of the process, the abatement of the lipid component of colostrum is accomplished using the centrifugal force. Since the separation efficiency increases with the temperature, the skimming is performed at 34-36°C.

The process consists in placing 250 Kg of frozen bovine colostrum in a 500 liter steel reactor, equipped with agitation and thermostat control, where it is diluted with 250 liters of aqueous isotonic solution (or demineralized water). This suspension is brought very quickly to a temperature of 34-36°C.

After reaching this temperature, the suspension is passed through a skimmer centrifuge suitable for effective skimming of the diluted colostrum, and the lean part is transferred to a second refrigerated 500 liter steel reactor where it is quickly brought to the temperature of 6-8°C. This temperature will be maintained for all subsequent processing steps.

### Casein separation

Caseins are hydrophobic, therefore in an aqueous solution they tend to gather together forming aggregates called micelles. What is important for our separation process are the dimensions of these micelles, that several studies have shown to be around 0.15 µm.

In the casein separation process, 0.1 µm ceramic filters were then used to remove the casein without having to resort to pH changes that could inactivate the delicate colostrum components. To avoid fouling phenomena, that is clogging of the filters, it is useful to use pre-filters having the size of 1.5 µm and 0.5 µm

### Diafiltration (dialysis) using a 3000 Da polymeric membrane

In this case, the capacity of the ultrafiltration membrane, having a molecular cut-off of 3000 Da, to allow the passage of the lactose in the permeate and to retain the protein component in the retentate is exploited.

The permeate from the 0.1 µm ceramic tangential filtration is concentrated about 10-20 times using a 3000 Da polymeric membrane, at a temperature of about 6-8°C. The retentate, with a content of about 10% of dry substance, is diafiltrated against demineralized water until the desired content of residual lactose is obtained.

### Sterilizing filtration using 0.45-0.22 µm, and subsequently 0.1 µm, filters

The solution is filtrated through a Sartorius Sartobran P cartridge filter (or other filter with similar characteristic) with a porosity of 0.45-0.22 µm, and subsequently with a 0.1 µm filter, and the filtered product is directly introduced in the collection container for rapid freezing in a nitrogen atmosphere, or for lyophilization.

### Drum filling and freezing

The filtered product is introduced in "*bag in box*" with a thickness of about 3 cm in a nitrogen atmosphere, and rapidly frozen at a temperature of -20°C .

### Lyophilization

The product is subjected to a lyophilization process, starting from a temperature of -50°C and ending at a temperature of 25-30°C.

### In Process Controls

At the end of the skimming phase, the residual fat content, that results to be on average between 1 and 2%, is controlled.

At the end of the ultrafiltration phase using a 3000 Da polymeric membrane, the dry residue in the retentate, that must be about 10%, and the lactose content, that must be less than 1% of the dry weight, are controlled.

### Process Yield

The yield of the process is evaluated by comparing the total content of dry matter in the retentate from the 3000 Da ultrafiltration to the initial weight of the frozen colostrum. On average, product yields between 5 and 7% are obtained.

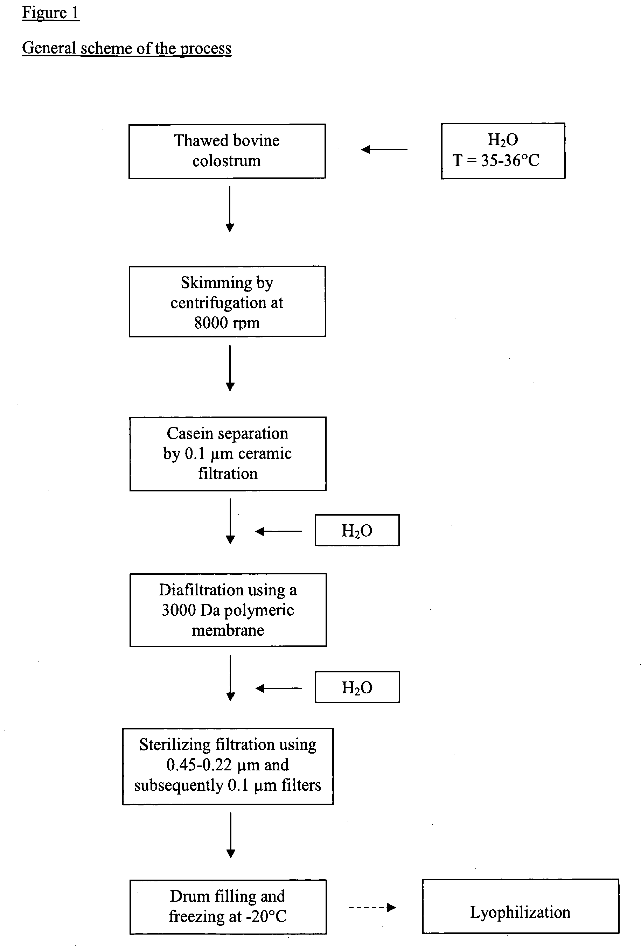

The general scheme of the process is described in Figure 1.

### Process of IqG depletion

### Materials and methods for IgG depletion

The IgG purification was carried out using a FPLC system (ÄKTA prime plus, GE Healthcare line-up) operating at low pressure (max 1MPa with a 0.1-50ml/min flow rate) equipped with a rotating fraction collector, an online UV detector (280 nm), a conductivity meter (0.001-999.9 mS/cm). The FPLC system was interfaced with PrimeView software. The chromatographic column was prepared by loading 400 ml of Protein G Sepharose 4 Fast Flow resin (GE Healthcare), in a HiScale™ 50 column.

The Protein G Sepharose 4 Fast Flow matrix contains, as a stationary phase, recombinant protein G immobilized on sepharosem using cyanogen bromide. The total IgG binding capacity of the column is equal to 23mg/ml of medium. Taking this parameter into account, and the IgG concentration in colostrum know in the literature, the starting sample was prepared starting from lyophilic colostrum at a concentration and volume such as not to saturate the binding sites of the column.

The chromatographic method involves three steps: binding, elution, and column regeneration.

The binding phase involves the activation of the column with 5 volumes of buffer A (Binding Buffer: Sodium phosphate 20mM pH 7) at a flow rate of 20ml/min, and the loading of the sample at the same flow. The eluate is monitored at 280 nm, and the fractions characterized by a significant UV absorption are collected by an automatic collector (15 ml per fraction).

The elution phase consists in the elution of IgG using a mobile phase acidified to pH 2.7 by using glycine hydrochloride, and maintained at a flow of 20 ml/min. After the elution phase is ended, 20% EtOH is infused for a proper preservation of the column (4°C) so as to preserve the resin from any molds contamination. The protein pattern of each collected fraction was analyzed by SDS-PAGE.

The IgG depleted fraction was subjected to desalinization and concentration by tangential diafiltration using 3 KDa hollow fibers (Hollowfiber; KrossFlo - Spetrum), and a subsequent lyophilization. The IgG content of the total product fraction and of the depleted fractions was determined by ELISA and electrophoretic analysis.

### 1. Evaluation of depletion process reproducibility

The described method was applied to 5 different samples, and the reproducibility was determined by calculating the percentage of depletion for each sample with respect to the content present in the mixture.

In the following, the % depletion results for each individual sample are reported: 94.88; 93.43; 93.21; 91.83; 93.71
Average: 93.41%± 1.09 (standard deviation)
Coefficient of variation (CV%) : 1.17%

Therefore, the reproducibility of the depletion process is considered to be satisfactory.

This method allows the elimination from the product of a portion higher than 90% of bovine immunoglobulins, and this is sufficient for many uses, such as the *in vitro* growth of cell cultures, but it is not sufficient from a regulatory point of view for an injection therapy. Therefore, to these steps an affinity chromatography system was added, that allows the complete elimination of immunoglobulins and their fragments.

### METHOD

The aim of the proposed method is the complete depletion of the immunoglobulin component from the mixture of biological factors described in the present invention, in particular of IgG, IgA e IgM subclasses, so as to obtain a formulation free of immunogenicity.

The process involves the following stages:
1) depletion of IgG and IgA by affinity chromatography;
2) depletion of IgM and aggregates of immunoglobulin nature or other nature, by tangential filtration, and using membranes having a molecular cut-off of 750 kDa/0.1µm;
3) immunoaffinity chromatography (HiTrap NHS-activated);
4) desalinization and concentration by diafiltration using membranes having a cut-off of 3/5kDa;
5) lyophilization.

The process was devised in view of an industrial application. All the stages of the process are transferable in view of a large scale purification process.

### 1. Depletion of IgG and IgA by affinity chromatography.

To date, the most widely used techniques for molecular recognition of antibodies are based on affinity chromatography based on a stationary phase of protein nature, isolated from bacterial surface or of recombinant nature, with a high selectivity towards immunoglobulins, such as Protein G from Streptococcus (IgG depletion) and Peptide M derived from Streptococcus Protein M (IgA depletion).

Such proteins are usually immobilized on preparative chromatographic columns, and they are able to bind immunoglobulins in a single pass with a high degree of purity and recovery.

The first stage of the process consists in the development of a depletion process based on affinity chromatography, and the use of Protein G and Peptide M as stationary phase capable of specific binding to the FC portion of IgG and IgA immunoglobulins.

After performing multiple affinity tests, we have provided for a scale-up of the IgG depletion method using a HiScale™ 50 column packed with 400ml of protein G Sepharose 4 Fast Flow (GE Healthcare) that consists of Protein G of recombinant origin immobilized on sepharose using cyanogen bromide (CNBr).

The binding capacity of the stationary phase is of 23 mg of IgG per ml, for a total of about 1g. The IgA depletion involves the use of a XK16 Column (GE Healthcare) packed with a Peptide M/Agarose (InvivoGen) stationary phase, wherein the recombinant Protein M is coupled to beads by means of a chemical resistance able to provide a support with a minimal non-specific binding.

In both columns the mobile phase was eluted using a FPLC system (AKTAprime plus, GE Healthcare) and monitored by a UV detector set at 280 nm, by a conductivity meter (0.001-999.9 mS/cm) and a pH meter.

During the binding phase, the immunoglobulins bind to the stationary phase, while the other proteins are eluted from the column. The IgG/A depleted proteins are automatically collected by UV monitoring the eluate. The next step consists in the elution of immunoglobulins using an acidic mobile phase at pH 2.5.

To ensure the sterility of the sample, between the IgG and IgA depletion steps, the eluted sample is filtered using 0.22 µm Stericup filters.

### 2. IgM and aggregates depletion by tangential filtration.

The IgM and aggregates depletion was performed by ultrafiltration, since these molecular species are characterized by a much higher molecular weight compared to the bioactive component. More in detail, the pentamers of immunoglobulins (IgM), which have on average a molecular weight of 800-900kDa, may be removed by tangential filtration by using membranes of appropriate size, while proteins with a lower molecular weight are able to cross it without been retained. Various filtration methods, as well as a variety of filters, were tested, and the best results in terms of depletion of IgM and aggregates were obtained by a tangential filtration system coupled to hollow fibers (cross flow filtration cartridges) with a cut-off of 750kDa/0.1 m; these include a feeding port, a retentate port, two permeate ports and a peristaltic pump for the recirculation of the sample

### 3. Immunoaffinity chromatography

The immunoaffinity chromatography step is performed using HiTrap NHS-activated columns.

### 4. Desalinization and concentration bv diafiltration

The desalinization and concentration step is performed using common diafiltration methods.

### 5. Lyophilization if needed.

### BIBLIOGRAPHIC REFERENCES

1. Gallina C, Turinetto V, Giachino C. A New Paradigm in Cardiac Regeneration: The Mesenchymal Stem Cell Secretome. Stem Cells Int. 2015;2015:765846. doi:10.1155/2015/765846. Epub 2015 May 5.
2. Bara JJ, Turner S, Roberts S, Griffiths G, Benson R, Trivedi JM, Wright KT. High content and high throughput screening to assess the angiogenic and neurogenic actions of mesenchymal stem cells in vitro. Exp Cell Res. 2015 Apr10;333(1):93-104.
3. Gazdhar A, Grad I, Tamò L, Gugger M, Feki A, Geiser T. The secretome of induced pluripotent stem cells reduces lung fibrosis in part by hepatocyte growth factor. Stem Cell Res Ther. 2014 Nov 10;5(6):123.
4. Kober J, Gugerell A, Schmid M, Zeyda M, Buchberger E, Nickl S, Hacker S,Ankersmit HJ, Keck M. Wound Healing Effect of Conditioned Media Obtained From Adipose Tissue on Human Skin Cells: A Comparative in Vitro Study. Ann Plast Surg.2014 Sep 30. [Epub ahead of print] PubMed PMID: 25275476.
5. Paschalidis T, Bakopoulou A, Papa P, Leyhausen G, Geurtsen W, Koidis P. Dental pulp stem cells' secretome enhances pulp repair processes and compensates TEGDMA-induced cytotoxicity. Dent Mater. 2014 Dec;30(12):e405-18. doi:10.1016/j.dental.2014.08.377. Epub 2014 Sep 18. PubMed PMID: 25241918.
6. Stoddart MJ, Bara J, Alini M. Cells and secretome--towards endogenous cell reactivation for cartilage repair. Adv Drug Deliv Rev. 2015 Apr;84:135-45. doi: 10.1016/j.addr.2014.08.007. Epub 2014 Aug 29. Review. PubMed PMID: 25174306.
7. De Lisio M, Jensen T, Sukiennik RA, Huntsman HD, Boppart MD. Substrate and strain alter the muscle-derived mesenchymal stem cell secretome to promotemyogenesis. Stem Cell Res Ther. 2014 Jun 6;5(3):74. doi: 10.1186/scrt463. PubMed PMID: 24906706; PubMed Central PMCID: PMC4097833.
8. Di Santo S, Seiler S, Fuchs AL, Staudigl J, Widmer HR. The secretome of endothelial progenitor cells promotes brain endothelial cell activity through PI3-kinase and MAP-kinase. PLoS One. 2014 Apr 22;9(4):e95731. doi:10.1371/journal.pone.0095731. eCollection 2014. PubMed PMID: 24755675; PubMedCentral PMCID: PMC3995762.
9. Maguire G. Stem cell therapy without the cells. Commun Integr Biol. 2013 Nov1;6(6):e26631. doi: 10.4161/cib.26631. Epub 2013 Sep 27. PubMed PMID: 24567776; PubMed Central PMCID: PMC3925653.
10. Drago D, Cossetti C, Iraci N, Gaude E, Musco G, Bachi A, Pluchino S. The stem cell secretome and its role in brain repair. Biochimie. 2013 Dec;95(12):2271-85. doi: 10.1016/j.biochi.2013.06.020. Epub 2013 Jul 1. Review. PubMed PMID:23827856; PubMed Central PMCID: PMC4061727.
11. Kapur SK, Katz AJ. Review of the adipose derived stem cell secretome. Biochimie. 2013 Dec;95(12):2222-8. doi: 10.1016/j.biochi.2013.06.001. Epub 2013Jun 14. Review. PubMed PMID: 23770442.
12. Zimmerlin L, Park TS, Zambidis ET, Donnenberg VS, Donnenberg AD. Mesenchymal stem cell secretome and regenerative therapy after cancer. Biochimie. 2013 Dec; 95(12):2235-45. Doi. 10.1016/j.biochi.2013.05.010. Epub 2013 Jun 5. Review PubMed PMID: 23747841; PubMed Central PMCID: PMC3825748.
13. Jayaraman P, Nathan P, Vasanthan P, Musa S, Govindasamy V. Stem cells conditioned medium: a new approach to skin wound healing management. Cell Biol.Int. 2013 Oct;37(10):1122-8. doi: 10.1002/cbin.10138. Epub 2013 Jun 24. PubMedPMID: 23716460.
14. Maumus M, Jorgensen C, Noël D. Mesenchymal stem cells in regenerative medicine applied to rheumatic diseases: role of secretome and exosomes. Biochimie. 2013 Dec;95(12):2229-34. doi: 10.1016/j.biochi.2013.04.017. Epub 2013 May 16. Review. PubMed PMID: 23685070.
15. WO2011/064114.
16. WO2013/098331.
17. WO2013/98333.

## Claims

1. A method for obtaining a mixture of biological factors isolated from colostrum, comprising the following steps:
a. skimming of colostrum, previously diluted with water or saline solution;
b. casein separation;
c. diafiltration using a polymeric membrane;
d. sterilizing filtration,
**characterized in that** the skimming step a) is performed by centrifugation at 5,000-10,000 rpm, preferably at about 8,000 rpm, at a temperature comprised between 35 and 36°C under stirring and the casein separation step b) is performed using 0.1 µm ceramic filters, optionally preceded by 1.5 µm and 0.5 µm pre-filters.

2. The method according to claim 1, **characterized in that** the diafiltration step c) is performed using a polymeric membrane having a cut-off of 2,000-5,000 Da, preferably of about 3,000 Da.

3. The method according to claim 1, **characterized in that** the retentate obtained from the diafiltration step is subjected to a sterilizing filtration step d) performed using a filter with a porosity of 0.22-0.45 µm, and subsequently a filter with a porosity of about 0.1 µm.

4. The method according to any one of claims 1-3, **characterized in that** the product obtained from the sterilizing filtration step is subjected to a lyophilization step.

5. The method according to any of the preceding claims, **characterized in that** it comprises a further step of IgG depletion by affinity chromatography.

6. The method according to claim 5, **characterized by** a further step of desalinization and concentration of the depleted sample.

7. The method according to claim 6, **characterized in that** said concentrated sample is subjected to a lyophilization process.

8. The method according to claim 1, **characterized in that** it comprises the following further steps for the total depletion of immunoglobulins IgG, IgA and IgM from the mixture of active factors:
a) affinity chromatography;
b) tangential flux filtration;
c) immunoaffinity chromatography.

9. The method according to claim 8, **characterized in that** the step c) is followed by a step of desalinization and concentration of the depleted sample by diafiltration using membranes having a cut-off of 3,000-5,000 Da.

10. The method according to any of the preceding claims, **characterized in that** the colostrum used originates from humans or domestic animals selected from the group consisting of bovines, ovines, and equines, preferably bovines.

11. The method according to claim 10, **characterized in that** the colostrum used is collected during the first hours after birth, preferably after six hours.

12. Combination of active factors obtained by the method of claim 1, comprising cytokines, growth factors, chemotactic factors, stem cell stimulating factors, complement proteins, antibacterial and/or antiviral factors, and immunoglobulins, **characterized in that** it contains lactose in an amount of less than 1%, and wherein the step c) of diafiltration is made using a polymeric membrane having a cut-off of about 3000 Da.

13. Combination of active factors obtained by the method of claim 1, comprising cytokines, growth factors, chemotactic factors, stem cell stimulating factors, complement proteins, antibacterial and/or antiviral factors, **characterized in that** it contains lactose in an amount of less than 1%, and wherein the step c) of diafiltration is made using a polymeric membrane having a cut-off of about 3000 Da, and **characterized in that** it is free of immunoglobulins.

## Patentansprüche

1. Verfahren zur Gewinnung eines Gemisches biologischer, aus Colostrum isolierter Faktoren, welches die folgenden Schritte umfasst:
a. Skimmen von vorab mit Wasser oder Kochsalzlösung verdünntem Colostrum;
b. Abtrennung von Casein;
c. Diafiltration unter Verwendung einer Polymer-Membran;
d. Sterilfitration,
**dadurch gekennzeichnet, dass** der Skimm-Schritt a) durch Zentrifugation bei 5,000 - 10,000 Upm durchgeführt wird, vorzugsweise bei etwa 8,000 Upm, bei einer Temperatur von zwischen 35 und 36°C unter Rühren und dass der Casein-Abtrennungs-Schritt b) unter Verwendung von 0,1 µm Keramikfilter durchgeführt wird, wahlweise nach 1,5 µm und 0,5 µm Vor-Filter.

2. Verfahren nach Anspruch 1, **dadurch gekennzeichnet, dass** der Diafiltrations-Schritt c) unter Verwendung einer Polymer-Membran mit einem Cut-off-Wert von 2,000-5,000 Da, vorzugsweise etwa 3,000 Da durchgeführt wird.

3. Verfahren nach Anspruch 1, **dadurch gekennzeichnet, dass** das aus dem Diafiltrations-Schritt erhaltene Retentat einem Sterilisations-Filtrations-Schritt d) unter Verwendung eines Filters mit einer Porösität von 0,22-0,45 µm, und anschließend einem Filter mit einer Porösität von etwa 0,1 µm unterzogen wird.

4. Verfahren nach einem der Ansprüche 1-3, **dadurch gekennzeichnet, dass** the product obtained from the sterilizing filtration step is subjected to a lyophilization step.

5. Verfahren nach einem der vorstehenden Ansprüche, **dadurch gekennzeichnet, dass** es einen weiteren Schritt der IgG-Entfernung mittels Affinitätschromatographie umfasst.

6. Verfahren nach Anspruch 5, **gekennzeichnet durch** einen weiteren Schritt der Entsalzung und Konzentrierung der gereinigten Probe.

7. Verfahren nach Anspruch 6, **dadurch gekennzeichnet, dass** die konzentrierte Probe einem Lyophiliserungs-Schritt unterzogen wird.

8. Verfahren nach Anspruch 1, **dadurch gekennzeichnet, dass** es die weiteren Schritte zur totalen Entfernung der Immunoglobuline IgG, IgA und IgM aus dem Gemisch der aktiven Faktoren umfasst:
a) Affinitätschromatographie;
b) tangentiale Fluxfiltration;
c) Immunaffinitäts-Chromatographie.

9. Verfahren nach Anspruch 8, **dadurch gekennzeichnet, dass** dem Schritt c) ein Schritt der Entsalzung und Konzentration der gereinigten Probe mittels Diafiltration unter Verwendung von Membranen mit einem Cut-off-Wert von 3,000-5,000 Da folgt.

10. Verfahren nach einem der vorstehenden Ansprüche, **dadurch gekennzeichnet, dass** das verwendete Colostrum von Menschen oder Nutztieren herrührt, ausgewählt aus der Gruppe bestehend aus Rindern, Schafen und Pferden, vorzugsweise Rindern.

11. Verfahren nach Anspruch 10, **dadurch gekennzeichnet, dass** das verwendete Colostrum während der ersten Stunden nach der Geburt gewonnen wird, vorzugsweise nach sechs Stunden.

12. Kombination aktiver Faktoren erhalten mit dem Verfahren nach Anspruch 1, welches Zytokine, Wachstumsfaktoren, chemotaktische Faktoren, Stammzellen-stimulierende Faktoren, Komplement-Proteine, antibakterielle und/oder antivirale Faktoren, und Immunglobuline enthält, **dadurch gekennzeichnet, dass** sie Laktose in einer Menge von weniger als 1 % enthält, und worin der Schritt c) der Diafiltration unter Verwendung einer Polymer-membran mit einem Cut-off-Wert von etwa 3000 Da erfolgt.

13. Kombination aktiver Faktoren erhalten mit dem Verfahren nach Anspruch 1, welches Zytokine, Wachstumsfaktoren, chemotaktische Faktoren, Stammzellen-stimulierende Faktoren, Komplement-Proteine, antibakterielle und/oder antivirale Faktoren enthält, **dadurch gekennzeichnet, dass** sie Laktose in einer Menge von weniger als 1 % enthält, und worin der Schritt c) der Diafiltration unter Verwendung einer Polymermembran mit einem Cut-off-Wert von etwa 3000 Da erfolgt, und **dadurch gekennzeichnet, dass** sie keine Immunglobuline enthält.

## Revendications

1. Procédé d'obtention d'un mélange de facteurs biologiques isolés à partir de colostrum, comprenant les étapes suivantes :
a. un écrémage du colostrum, préalablement dilué avec de l'eau ou une solution saline ;
b. une séparation de la caséine ;
c. une diafiltration en utilisant une membrane polymère ;
d. une filtration stérile,
**caractérisé en ce que** l'étape d'écrémage a) est réalisée par une centrifugation à 5 000-10 000 tr/min, de préférence à environ 8 000 tr/min, à une température comprise entre 35 et 36 °C sous agitation, et l'étape de séparation de la caséine b) est réalisée en utilisant des filtres en céramique de 0,1 µm, facultativement précédés de préfiltres de 1,5 µm et 0,5 µm.

2. Procédé selon la revendication 1, **caractérisé en ce que** l'étape de diafiltration c) est réalisée en utilisant une membrane polymère possédant un seuil de coupure de 2 000-5 000 Da, de préférence d'environ 3 000 Da.

3. Procédé selon la revendication 1, **caractérisé en ce que** le rétentat obtenu à partir de l'étape de diafiltration est soumis à une étape de filtration stérile d) réalisée en utilisant un filtre possédant une porosité de 0,22-0,45 µm, et ensuite avec un filtre possédant une porosité d'environ 0,1 µm.

4. Procédé selon l'une quelconque des revendications 1 à 3, **caractérisé en ce que** le produit obtenu à partir de l'étape de filtration stérile est soumis à une étape de lyophilisation.

5. Procédé selon l'une quelconque des revendications précédentes, **caractérisé en ce qu'**il comprend une étape supplémentaire de déplétion des IgG par une chromatographie d'affinité.

6. Procédé selon la revendication 5, **caractérisé par** une étape supplémentaire de désalinisation et de concentration de l'échantillon ayant subi la déplétion.

7. Procédé selon la revendication 6, **caractérisé en ce que** ledit échantillon concentré est soumis à un processus de lyophilisation.

8. Procédé selon la revendication 1, **caractérisé en ce qu'**il comprend les étapes supplémentaires suivantes pour obtenir une déplétion totale des immunoglobulines IgG, IgA et IgM à partir du mélange de facteurs actifs :
a) une chromatographie d'affinité ;
b) une filtration à flux tangentiel ;
c) une chromatographie d'immunoaffinité.

9. Procédé selon la revendication 8, **caractérisé en ce que** l'étape c) est suivie d'une étape de désalinisation et de concentration de l'échantillon ayant subi la déplétion par une diafiltration en utilisant des membranes possédant un seuil de coupure de 3 000-5 000 Da.

10. Procédé selon l'une quelconque des revendications précédentes, caractérisé en ce le colostrum utilisé provient d'humains ou d'animaux domestiques sélectionnés dans le groupe consistant en des bovins, des ovins, et des équidés, de préférence des bovins.

11. Procédé selon la revendication 10, **caractérisé en ce que** le colostrum utilisé est collecté au cours des premières heures suivant la naissance, de préférence au bout de six heures.

12. Combinaison de facteurs actifs obtenue par le procédé selon la revendication 1, comprenant des cytokines, des facteurs de croissance, des facteurs chimiotactiques, des facteurs de stimulation des cellules souches, des protéines du complément, des facteurs antibactériens et/ou antiviraux, et des immunoglobulines, **caractérisée en ce qu'**elle contient du lactose en une quantité inférieure à 1 %, et dans laquelle l'étape c) de diafiltration est réalisée en utilisant une membrane polymère possédant un seuil de coupure d'environ 3 000 Da.

13. Combinaison de facteurs actifs obtenue par le procédé selon la revendication 1, comprenant des cytokines, des facteurs de croissance, des facteurs chimiotactiques, des facteurs de stimulation des cellules souches, des protéines du complément, des facteurs antibactériens et/ou antiviraux, **caractérisée en ce qu'**elle contient du lactose en une quantité inférieure à 1 %, et dans laquelle l'étape c) de diafiltration est réalisée en utilisant une membrane polymère possédant un seuil de coupure d'environ 3 000 Da, et **caractérisée en ce qu'**elle est dépourvue d'immunoglobulines.
